# EUROPEAN PATENT APPLICATION

(11) **EP 4 276 456 A1**
(43) Date of publication of application: **15.11.2023**
(21) Application number: 21917707.8
(22) Date of filing: 24.12.2021
(51) Int. Cl.: G01N 27/62, G01N 33/50

(54) **METHOD FOR TESTING POSSIBILITY OF HAVING CONTRACTED PROSTATE CANCER**

(30) Priority: 07.01.2021 JP 2021001676
(71) Applicant: SUMITOMO CHEMICAL COMPANY, LIMITED, Tokyo 103-6020 (JP); National Institute Of Advanced Industrial Science and Technology, Chiyoda-ku Tokyo 100-8921 (JP); Aino University, Ibaraki-shi, Osaka, 567-0011 (JP)
(72) Inventor: OZOE, Atsufumi, Osaka-shi, Osaka 554-8558 (JP); KOBAYASHI, Kentaro, Osaka-shi, Osaka 554-8558 (JP); TAKAHASHI, Yasuhiko, Osaka-shi, Osaka 554-8558 (JP); MATSUNAGA, Kohei, Osaka-shi, Osaka 554-8558 (JP); SATO, Takaaki, Ikeda-shi, Osaka 563-8577 (JP); MIZUTANI, Yoichi, Ibaraki-shi, Osaka 567-0011 (JP)
(74) Representative: Vossius & Partner Patentanwälte Rechtsanwälte mbB
(86) International application number: PCT/JP2021/048082
(87) International publication number: WO 2022/149487

(57) **Abstract**

The present invention provides a technology for performing a test for prostate cancer. This method is for testing the possibility of having prostate cancer, the method comprising (1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

## Description

### Technical Field

The present disclosure discloses a method for testing for the possibility of having prostate cancer.

### Background Art

Prostate cancer has a high incidence in men, with more than 100000 cases per year in Japan. With the westernization of diets in Japan, the aging of the population, etc., the incidence % of prostate cancer and the number of prostate cancer cases are expected to continue to increase in the future. As with other cancers, early detection and early treatment are important.

In prostate cancer testing, a screening test is performed using blood PSA levels (Prostate Specific Antigen) as an index. However, the PSA test is problematic because of a high number of false positives. There is room for improvement in the accuracy of prediction. On the other hand, a needle biopsy is also performed to check for the presence of cancer cells in prostate tissue by collecting prostate tissue. Although this test is used as a definitive diagnostic method, the test is highly invasive and carries the risk of causing infection.

Further, in recent years, various technologies have been reported to identify prostate cancer using the sense of smell of animals (e.g., Non-Patent Literature (NPL) 1).

### Citation List

### Non-patent Literature

NPL 1: Gordon, R.T., et al. The use of canines in the detection of human cancer. J. Altern. Complement. Med. 14, 61-67 (2008).

### Summary of Invention

### Technical Problem

An object of the present disclosure is to provide a prostate cancer testing technology.

### Solution to Problem

In view of the above problem, the present inventors conducted extensive research. As a result, the present inventors found that the presence of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone in a body fluid sample collected from a subject is a marker of prostate cancer. The present inventors conducted further research based on this finding and have accomplished the present invention.

Specifically, the present disclosure includes the following embodiments.
Item 1. A method for testing for the possibility of having prostate cancer, comprising the step of
   (1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item 2. The method according to Item 1, further comprising the step of
   (2a) determining that the subject has a high possibility of having prostate cancer when the amount or concentration of the biomarker detected in step (1) is more than or equal to a reference value A and/or
   (2b) determining that the subject has a low possibility of having prostate cancer when the amount or concentration of the biomarker detected in step (1) is less than or equal to a reference value B.
Item 3. The method according to Item 2, wherein the reference value A and the reference value B are values based on the maximum, average, percentile, or minimum value of the amount or concentration of the biomarker in body fluid samples collected from subjects determined to have no prostate cancer or subjects determined to have prostate cancer.
Item 4. The method according to any one of Items 1 to 3, wherein the measurement method in step (1) is gas chromatography-mass spectrometry.
Item 5. The method according to Item 4, wherein an ion monitored in the gas chromatography-mass spectrometry is:
   (A) at least one member selected from the group consisting of ions with m/z 129, ions with m/z 59, and ions with m/z 100 when dimethyl glutarate is measured;
   (B) at least one member selected from the group consisting of ions with m/z 121, ions with m/z 106, and ions with m/z 91 when 2,6-xylidine is measured;
   (C) at least one member selected from the group consisting of ions with m/z 105, ions with m/z 59, and ions with m/z 77 when 2-hydroxy-2-methylpropiophenone is measured;
   (D) at least one member selected from the group consisting of ions with m/z 163, ions with m/z 178, and ions with m/z 121 when 2,6-di(propan-2-yl)phenol is measured;
   (E) at least one member selected from the group consisting of ions with m/z 115, ions with m/z 55, ions with m/z 59, and ions with m/z 114 when dimethyl succinate is measured;
   (F) at least one member selected from the group consisting of ions with m/z 105, ions with m/z 77, and ions with m/z 120 when acetophenone is measured;
   (G) at least one member selected from the group consisting of ions with m/z 43, ions with m/z 121, and ions with m/z 77 when 2-phenyl-2-propanol is measured; and
   (H) at least one member selected from the group consisting of ions with m/z 138, ions with m/z 82, and ions with m/z 54 when 3,5,5-trimetyl-2-cyclohexenone is measured.
Item 6. The method according to any one of Items 1 to 5, wherein the biomarker is two or more biomarkers.
Item 7. The method according to any one of Items 1 to 6, wherein the body fluid sample is a urine sample.
Item 8. A prostate cancer biomarker in a body fluid sample, the biomarker comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item 9. A prostate cancer test drug comprising a standard sample and/or a detecting agent, the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

Further, the present disclosure includes the following as other embodiments.
Item A. A method for assisting in determining the possibility of having prostate cancer, comprising the step of
   (1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item B. A method for measuring a prostate cancer biomarker, comprising the step of
   (1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item C. A standard sample and/or a detecting agent for use as a prostate cancer test drug,
   the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
   the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item D. Use of a standard sample and/or a detecting agent in testing for prostate cancer,
   the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
   the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
Item E. Use of a standard sample and/or a detecting agent in producing a prostate cancer test drug,
   the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
   the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

### Advantageous Effects of Invention

The present disclosure can provide a biomarker-based prostate cancer testing technology.

### Brief Description of Drawings

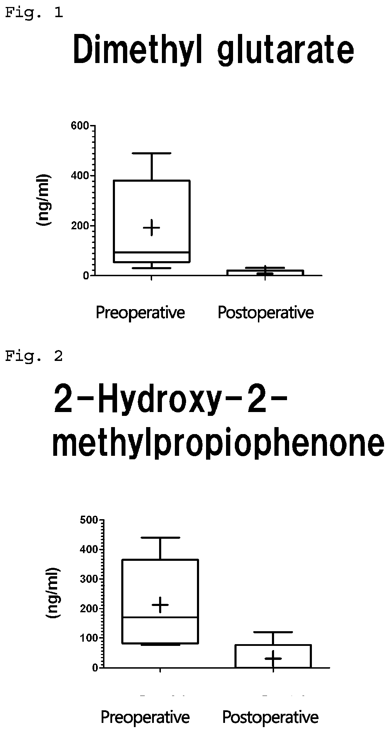

Fig. 1 shows boxplots representing the measurement results of dimethyl glutarate in Example 2. The ordinate shows urinary concentration of the target biomarker. On the abscissa, "Preoperative" shows the results of urine samples collected from prostate cancer patients before total prostatectomy, whereas "Postoperative" shows results of urine samples collected from prostate cancer patients after total prostatectomy; and the preoperative and postoperative results are results of the same group of subjects. In the boxplots, each box indicates the range of concentration distribution of the target biomarker in urine samples corresponding to 25-75% of all samples, and the range indicated by horizontal lines indicates the concentration distribution range of the target biomarker in urine samples corresponding to 10-90% of all samples. The horizontal bar in the boxes indicates the median concentration of the target biomarker in each population (preoperative and postoperative). The "+" in the boxes indicates the mean value.
Fig. 2 shows measurement results of 2-hydroxy-2-methylpropiophenone in Example 2. The ordinate, abscissa, bars, and dotted lines are defined in the same way as in Fig. 1.
Fig. 3 shows measurement results of 2,6-diisopropylphenol (2,6-di(propan-2-yl)phenol) in Example 2. The ordinate, abscissa, bars, and dotted lines are defined in the same way as in Fig. 1.
Fig. 4 shows measurement results of dimethyl succinate in Example 2. The ordinate, abscissa, bars, and dotted lines are defined in the same way as in Fig. 1.
Fig. 5 shows measurement results of acetophenone in Example 2. The ordinate, abscissa, bars, and dotted lines are defined in the same way as in Fig. 1.
Fig. 6 shows measurement results of 2-phenyl-2-propanol in Example 2. The ordinate, abscissa, bars, and dotted lines are defined in the same way as in Fig. 1.
Fig. 7 shows measurement results of 3,5,5-trimetyl-2-cyclohexenone. The ordinate, abscissa, bars, and dotted lines are defined in the same way as in Fig. 1.

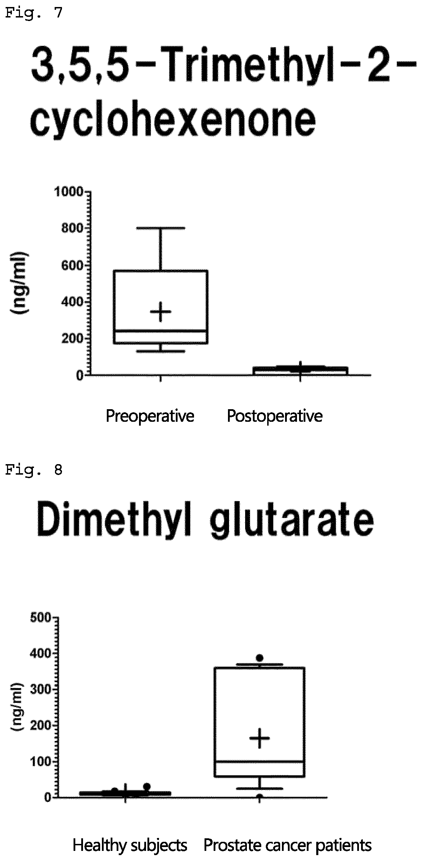

Fig. 8 shows boxplots representing the measurement results of dimethyl glutarate in Example 3. The ordinate shows urinary concentrations of the target biomarker. On the abscissa, "Healthy subjects" indicate the results of urine samples collected from healthy subjects, "Prostate cancer patients" indicate the results of urine samples collected from prostate cancer patients. In the boxplots, each box indicates the range of concentration distribution of the target biomarker in urine samples corresponding to 25-75% of all samples, and the range indicated by horizontal lines indicates the concentration distribution range of the target biomarker in urine samples corresponding to 10-90% of all samples. The horizontal bar in the boxes indicates the median concentration of the target biomarker in each population (preoperative and postoperative). The "+" in the boxes indicates the mean value.
Fig. 9 shows measurement results of 2-hydroxy-2-methylpropiophenone in Example 3. The ordinate, abscissa, bars, and dotted lines are defined in the same way as in Fig. 8.
Fig. 10 shows measurement results of 2,6-diisopropylphenol (2,6-di(propan-2-yl) phenol) obtained in Example 3. The ordinate, abscissa, bars, and dotted lines are defined in the same way as in Fig. 8.
Fig. 11 shows measurement results of dimethyl succinate in Example 3. The ordinate, abscissa, bars, and dotted lines are defined in the same way as in Fig. 8.
Fig. 12 shows measurement results of acetophenone in Example 3. The ordinate, abscissa, bars, and dotted lines are defined in the same way as in Fig. 8.
Fig. 13 shows measurement results of 2-phenyl-2-propanol in Example 3. The ordinate, abscissa, bars, and dotted lines are defined in the same way as in Fig. 8.
Fig. 14 shows measurement results of 3,5,5-trimetyl-2-cyclohexenone in Example 3. The ordinate, abscissa, bars, and dotted lines are defined in the same way as in Fig. 8.
Fig. 15 shows measurement results of 2,6-xylidine in Example 3. The ordinate, abscissa, bars, and dotted lines are defined in the same way as in Fig. 8.

### Description of Embodiments

In the present specification, the terms "comprising," "containing," and "including" include the concepts of comprising, containing, consisting essentially of, and consisting of.

In the present specification, the expression "and/or" encompasses the meanings of both "and" and "or." For example, the expression "A and/or B" encompasses the meanings of both "A and B" and "A or B."

### 1. Method for testing for the possibility of having prostate cancer

The present disclosure provides a method for testing for the possibility of having prostate cancer, comprising the step of
(1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone (which may be referred to herein as "the target biomarker") (this method may be referred to herein as "the testing method of the present disclosure").

The present disclosure relates to a biomarker for prostate cancer in a body fluid sample, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

These are described below.

### 1-1. Step (1)

The prostate cancer to be tested is not limited and encompasses prostate cancers of any type, any degree of severity (e.g., mild, moderate, severe), and any stage.

The subject refers to a target organism of the testing method of the present disclosure. The species of the subject is not limited. Examples of species of the subject includes various mammalian animals, such as humans, monkeys, mice, rats, dogs, cats, and rabbits, and preferably humans.

The prostate cancer status of the subject is not limited. Examples of the subject include subjects who are not known to have prostate cancer or be in good health, subjects with no history of prostate cancer, subjects with a history of prostate cancer treated for prostate cancer, and subjects already determined to have (or not have) prostate cancer by other testing methods (e.g., the PSA test). When the subject is a human, any person, regardless of previous medical history, including persons who appear to be healthy, can be the test target. For persons who appear to be healthy, the test is effective for early detection and diagnosis of prostate cancer in general health examinations and comprehensive physical examinations. For subjects who are suspected to have prostate cancer, the testing method of the present disclosure can assist in the diagnosis of prostate cancer.

The body fluid sample is not limited as long as it can contain the target biomarker. Examples of the body fluid sample include body fluids such as blood that has passed through prostate cancer tissue, body fluids produced from such body fluids, and samples derived from such body fluids. Examples of usable samples derived from body fluids include samples obtained by purification of body fluids. The purification treatment is not limited as long as the amount or concentration of the target biomarker is not significantly reduced. Examples include treatments for removal of salts, proteins, etc. (e.g., enzymatic treatment, chromatographic column purification treatment, centrifugal separation treatment, and like treatment). Specific examples of body fluid samples include urine, blood, cerebrospinal fluid, body secretions, saliva, and sputum; and samples derived from these body fluids. Among these, urine samples (urine and urine-derived samples) are preferred from the viewpoint of testing efficiency. The method of collecting body fluids is not limited and can be, for example, in accordance with or pursuant to the method used in general periodic health examinations. The timing of the collection of body fluids is not limited and can be, for example, first thing in the morning, in the evening, before bed, and after or before meals. The body fluid sample can be used immediately after collection or preparation or can also be used after being cryopreserved. Since the amount of 2,6-di(propan-2-yl)phenol decreases at room temperature due to oxidative denaturation and 2,6-di(propan-2-yl)phenol is thus considered to have a half-life of 3 to 12 hours at room temperature, samples that have been left at room temperature for half a day or more are not suitable for use. The cryopreservation method is not limited. For example, the samples can be placed in a sealed glass container, frozen as soon as possible (e.g., at about -80°C), and stored at a general freezer temperature (e.g., about -20°C). The storage period is not limited and can be one year or longer, but is preferably a shorter period. Body fluid samples may be used singly or in a combination of two or more.

The target biomarker is at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenon. Table 1 shows the structural formulas of these target biomarkers.

**Table 1**

| **Compound name** | **Structure** |
|---|---|
| **Dimethyl glutarate** | 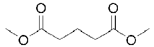 |
| 2,6-Xylidine | 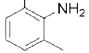 |
| 2-Hydroxy-2-methylpropiophenone | 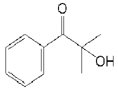 |
| 2,6-di(propan-2-yl)phenol | 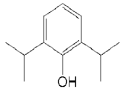 |
| Dimethyl succinate | 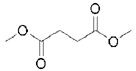 |
| Acetophenone | 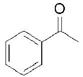 |
| 2-Phenyl-2-propanol | 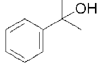 |
| 3,5,5-Trimetyl-2-cyclohexenone | 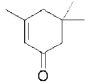 |

The target biomarker to be measured in step (1) can be only one type of biomarker. Alternatively, 2 or more, 3 or more, 4 or more, 5 or more, 6 or more, 7 or more, or 8 types of the biomarkers can be used as the target bomarkers. Combining more target biomarkers enables more accurate testing for prostate cancer etc.

The method of measuring the amount or concentration of the target biomarker is not limited as long as the method is capable of detecting the target biomarker. From the viewpoint of detection sensitivity etc., the method is preferably a mass spectrometry (MS) method, and more preferably a gas chromatography mass spectrometry (GC/MS) method.

The GC/MS method is performed by separating substances in a body fluid sample of a subject by gas chromatography and analyzing each separated fraction of a substance by mass spectrometry.

Gas chromatography can be performed in accordance with or pursuant to a known method. The vaporization and introduction of a body fluid sample in gas chromatography can be performed by selecting an appropriate method from the known methods, depending on the conditions of the body fluid sample. For example, gas chromatography can be performed by adsorbing the components of a body fluid sample onto a volatile organic substance-adsorbing material, including a general-purpose solid-phase microextraction fiber, followed by heating and desorption. The volatile organic substance-adsorbing material is not limited and examples include silicone resins, and more preferably silicone elastomers. Examples of silicone resins and silicone elastomers include polydimethylsiloxane (PDMS) or adsorbents made of a modified silicone compound in which the methyl group of the polymer chain in the silicone compound is replaced with a vinyl group or a phenyl group. The volatile organic substance-adsorbing material can be used in combination with a suitable carrier.

The carrier gas in gas chromatography is not limited as long as it is an inert gas. Examples of carrier gases include helium, nitrogen, and argon, and preferably helium. The stationary phase of gas chromatography is not limited. Examples of the stationary phase include polyethylene glycol, polymethylcyanoalkylsiloxane, polydimethylsiloxane/diphenylsiloxane, and polydimethylsiloxane. Among these, stationary phases with higher polarity are preferable. Specific examples include preferably polyethylene glycol, polymethylcyanoalkylsiloxane, and polydimethylsiloxane/diphenylsiloxane, more preferably polyethylene glycol and polymethylcyanoalkylsiloxane, and even more preferably polyethylene glycol. Other examples of stationary phases include silica gel, activated carbon, zeolite, and activated alumina. The detector for gas chromatography is not limited as long as it is capable of detecting a wide variety of components. Examples of detectors include thermal conductivity detectors (TCD), hydrogen flame ionization detectors (FID), and electron capture detectors (ECD).

Mass spectrometry can be performed in accordance with or pursuant to a known method. The ionization method in mass spectrometry is not limited. Examples of the ionization method include electron ionization (EI), positive chemical ionization (PCI), and negative chemical ionization (NCI), and like methods, and preferably the EI method. The ionization voltage in the EI method is not limited, but is typically about 70 V. The method for separating ionized sample molecules in mass spectrometry is not limited. Examples of the separation method include time-of-flight mass spectrometry, magnetic field polarized spectrometry, quadrupole spectrometry, ion trap spectrometry, Fourier transform ion cyclotron resonance spectrometry, and tandem spectrometry.

The amount or concentration of the target biomarker can be determined by quantifying the amount of ion indicating the target biomarker obtained by mass spectrometry. For example, the amount or concentration of the target biomarker can be calculated based on the amplitude value or area of the measured peak of the ion in the mass spectrum obtained by mass spectrometry. When the amplitude value or area of the measured peak is used, a treatment for reducing background can also be additionally performed, if necessary. Examples of monitored ions used for quantification include the following monitor ions:
(A) when dimethyl glutarate is measured, the monitored ion is at least one member selected from the group consisting of ions with m/z 129, ions with m/z 59, and ions with m/z 100;
(B) when 2,6-xylidine is measured, the monitored ion is at least one member selected from the group consisting of ions with m/z 121, ions with m/z 106, and ions with m/z 91;
(C) when 2-hydroxy-2-methylpropiophenone is measured, the monitored ion is at least one member selected from the group consisting of ions with m/z 105, ions with m/z 59, and ions with m/z 77;
(D) when 2,6-di(propan-2-yl)phenol is measured, the monitored ion is at least one member selected from the group consisting of ions with m/z 163, ions with m/z 178, and ions with m/z 121;
(E) when dimethyl succinate is measured, the monitored ion is at least one member selected from the group consisting of ions with m/z 115, ions with m/z 55, ions with m/z 59, and ions with m/z 114;
(F) when acetophenone is measured, the monitored ion is at least one member selected from the group consisting of ions with m/z 105, ions with m/z 77, and ions with m/z 120;
(G) when 2-phenyl-2-propanol is measured, the monitored ion is at least one member selected from the group consisting of ions with m/z 43, ions with m/z 121, and ions with m/z 77; and
(H) when 3,5,5-trimetyl-2-cyclohexenone is measured, the monitored ion is at least one member selected from the group consisting of ions with m/z 138, ions with m/z 82, and ions with m/z 54.

In one preferred embodiment of the present disclosure, the underlined ion species among the above monitor ion species can be used as quantitation ions, and the other ion species can be used as reference ion species.

Examples of the method for measuring the amount or concentration of the target biomarker include, in addition to the MS method, the surface plasmon resonance method and other detection methods using, for example, metal oxide semiconductors, conductive polymers, polymer thin films, cholesteric liquid crystals, Schottky diodes, MOSFETs, thermistors, varistors, thermocouples, quartz crystal oscillators, SAW devices, fluorescent substances, light-absorbing substances, or olfactory sensory cells.

The testing method comprising step (1) according to the present disclosure can provide a measurement value for calculating an index for determining the possibility of having prostate cancer, and preferably further provide an index for determining the possibility of having prostate cancer. This can assist in, for example, determining the possibility of having prostate cancer.

The target biomarker can also be used in various aspects of prostate cancer testing. The target biomarkers can be used not only for screening for prostate cancer in physical examinations etc., but also for monitoring therapeutic effects during or after prostate cancer treatment, detecting recurrence after prostate cancer treatment, and determining patient prognosis.

### 1-2. Step (2)

In one embodiment, the testing method of the present disclosure preferably further comprises the step of
(2a) determining that the subject has a high possibility of having prostate cancer when the amount or concentration of the biomarker detected in step (1) is equal to or higher than a reference value A; and/or the step of
(2b) determining that the subject has a low possibility of having prostate cancer when the amount or concentration of the biomarker detected in step (1) is equal to or lower than a reference value B (these steps (2a) and (2b) may be collectively referred to as "step (2)"). Here, the "possibility of having prostate cancer" means "possibility of having prostate cancer at the time of collecting a body fluid sample."

According to the testing method of the present disclosure comprising the step (2), the possibility of having prostate cancer can be determined. Since the testing method of the present disclosure can determine the possibility of having prostate cancer with high accuracy, the testing method of the present disclosure comprising step (2) can more reliably determine a subject with prostate cancer as having "prostate cancer (or having no prostate cancer)" (specifically, the possibility of erroneously determining that "the subject does not have prostate cancer (or the subject has prostate cancer)" can be reduced).

The reference values (reference value A and reference value B) may be individually set as appropriate by a person skilled in the art in terms of decision sensitivity, decision specificity, positive hit rate, negative hit rate, etc. The reference values can be either values set on a case-by-case basis or pre-set values according to race, age, etc. The reference value can be, for example, a value based on the maximum, average, percentile, or minimum amount or concentration of the target biomarker in body fluid samples taken from subjects determined to have no prostate cancer or from subjects determined to have prostate cancer.

A "reference value" is a value such that when the presence or absence of disease onset is determined based on that value, the determination sensitivity (true positive rate) and determination specificity (true negative rate) both show sufficiently high values. For example, a value that shows a high positive rate in individuals having prostate cancer and a high negative rate in individuals not having prostate cancer can be set as a reference value.

In the present specification, the "determination sensitivity" refers to the percentage of positive (abnormal) values (the percentage of true positives) when the test is performed in a population that has a specific disease. The "determination specificity" refers to the percentage of negative (normal) values (the percentage of true negatives) when the test is performed in a population not having the specific disease. The "positive predictive value" refers to the percentage of individuals who actually have the disease among the subjects who tested positive. The "negative predictive value" refers to the percentage of individuals who do not actually have the disease among the subjects who tested negative.

The method of setting a reference value is well known in this technical field. More specifically, for example, the amount or concentration of the target biomarker in biological samples taken from subjects who have been determined to have no prostate cancer or subjects who have been determined to have prostate cancer is measured. Using the measured values, a statistical analysis based on, for example, the analysis of a receiver operating characteristic (ROC) curve (more specifically, for example, a method using Youden's index) is performed to set a reference value.

Such a reference value does not take a specific value, but rather varies depending on the sample population used in setting the reference value.

Even when the same subject is used as a target, the measured value may vary depending on the analysis method used. Therefore, the reference value is set according to the analysis method used.

The reference value can also be set based on the measured value of a body fluid sample taken from the same subject before a certain period of time. The "certain period of time" is not limited as long as it is a period of time during which the measured value can vary within the same subject. The "certain period of time" can be, for example, a period ranging from 1 month to 10 years, 2 months to 5 years, 3 months to 2 years, or 4 months to 1 year. If the subject is under treatment and/or undergoing surgery, the reference value can also be set based on the measured value of a body fluid sample taken before the treatment and/or surgery.

### 2. Diagnosis of prostate cancer with greater accuracy

When a subject is determined to have a high possibility of having prostate cancer by the testing method of the present disclosure comprising step (2a), a combination of the testing method of the present disclosure with step (3) of applying a prostate cancer diagnosis method to the subject who has been determined to have a high possibility of having prostate cancer in step (2a) can more accurately diagnose prostate cancer. Since the testing method of the present disclosure can determine the possibility of having prostate cancer with a high degree of accuracy, a combination of the testing method of the present disclosure with step (3) can more reliably diagnose a subject truly having prostate cancer as "having prostate cancer" (in other words, the possibility of determining that the subject having no prostate cancer is the target of diagnosis in step (3), the possibility of misdiagnosing a subject having prostate cancer as "having no prostate cancer," and the possibility of excluding a subject having prostate cancer from the target of diagnosis in step (3) can be further reduced).

The method for diagnosing prostate cancer applied in step (3) is not limited, and various known diagnostic methods can be used. Examples of diagnostic methods include rectal palpation, ultrasonography, MRI scan, CT scan, and needle biopsy. Such diagnostic methods can be used singly or in a combination of two or more.

### 3. Treatment of prostate cancer

When a subject is determined to have high possibility of having prostate cancer comprising step (2a) according to the testing method of the present disclosure or a subject is diagnosed as prostate cancer in step (3), prostate cancer in the subject can be treated by subjecting the subject determined to have prostate cancer in step (2a) or the subject diagnosed as having prostate cancer in step (3) to a prostate cancer treatment step (step (4)). Further, since the testing method of the present disclosure can determine the possibility of having prostate cancer with high sensitivity, a further combination of step (4) with either the testing method of the present disclosure alone or a combination of the testing method of the present disclosure and step (3) can more reliably treat a subject truly having prostate cancer (i.e., the possibility of excluding a subject truly having prostate cancer from the target of treatment can be reduced).

The method for treating prostate cancer is not limited and various known treatment methods can be used. Examples of treatment methods include chemotherapy, surgical treatment methods, radiation therapy methods, and immunotherapy. These can be performed according to known methods.

The therapeutic agent for use in chemotherapy is not limited and various anticancer agents can be used. Examples of anticancer agents include alkylating agents, antimetabolites, microtubule inhibitors, antibiotic anticancer agents, topoisomerase inhibitors, platinum agents, molecular target drugs, hormonal agents, and biologics. Examples of alkylating agents include cyclophosphamide, ifosfamide, nitrosourea, dacarbazine, temozolomide, nimustine, busulfan, melphalan, procarbazine, and ranimustine. Examples of antimetabolites include enocitabine, carmofur, capecitabine, tegafur, tegafur-uracil, tegafur-gimeracil-oteracil potassium, gemcitabine, cytarabine, cytarabine octophosphate, nelarabine, fluorouracil, fludarabine, pemetrexed, pentostatin, methotrexate, cladribine, doxyfluridine, hydroxycarbamide, and mercaptopurine. Examples of microtubule inhibitors include alkaloid anticancer agents such as vincristine, and taxane anticancer agents such as docetaxel and paclitaxel. Examples of antibiotic anticancer agents include mitomycin C, doxorubicin, epirubicin, daunorubicin, bleomycin, actinomycin D, aclarubicin, idarubicin, pirarubicin, peplomycin, mitoxantrone, amrubicin, and zinostatin stimalamer. Examples of topoisomerase inhibitors include CPT-11, irinotecan, and nogitecan, which are topoisomerase I inhibitors, and etoposide and sobuzoxane, which are topoisomerase II inhibitors. Examples of platinum preparations include cisplatin, nedaplatin, oxaliplatin, and carboplatin. Examples of hormonal agents include dexamethasone, finasteride, tamoxifen, astrozole, exemestane, ethinylestradiol, chlormadinone, goserelin, bicalutamide, flutamide, prednisolone, leuprorelin, letrozole, estramustine, toremifene, fosfestrol, mitotane, methyltestosterone, medroxyprogesterone, and mepitiostane. Examples of biologics include interferon α, interferon β, interferon γ, interleukin 2, ubenimex, and dried BCG. Examples of molecular target agents include nivolumab, pembrolizumab, rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tosituzumab, bortezomib, gemtuzumab ozogamicin, ibritumomab ozogamicin, ibritumomab tiuxetan, tamibarotene, and tretinoin. Examples of molecular target drugs include, in addition to those specified herein, inhibitors targeting angiogenesis such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and vascular endothelial growth factor receptor 2 inhibitors (α-VEGFR-2 antibody); various tyrosine kinase inhibitors such as MAP kinase inhibitors, inhibitors targeting cytokines, proteasome inhibitors, antibody-anticancer drug combinations, and like molecular target drugs. These inhibitors include antibodies. Therapeutic agents may be used singly or in a combination of two or three or more.

### 4. Prostate cancer test drug

In one embodiment, the present disclosure relates to a prostate cancer test drug containing a standard sample and/or a detection agent,
the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone (the prostate cancer test drug may be referred to herein as the "test drug of the present disclosure"). This is described below.

The standard sample is not limited as long as it is at least one compound selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimethyl-2-cyclohexenone, or a modified product thereof. The standard sample can be used, for example, to create a standard curve used in quantifying the target biomarker. The standard sample is a known compound or can be obtained from a known compound by an existing compound modification method.

The detection agent is not limited as long as it can be used to detect the target biomarker. When the target biomarker has a structure (e.g., an atomic group) that develops color when converted into another compound by a chemical reaction, a substance that causes the chemical reaction (a color-developing reagent) can be used as a detection agent. Examples of coloring reagents include phosphomolybdic acid, anisaldehyde, ammonium cerium molybdate, sulfuric acid, ninhydrin, Dragendorff, dinitrophenylhydrazine, palladium chloride, basic potassium permanganate, bromocresol green, and iron chloride (II). Other examples of usable detecting agents include antibodies against compounds that constitute a target biomarker, and detection tags that aid detection of a target biomarker by mass spectrometry.

The test drug of the present disclosure may be in the form of a composition containing a standard sample and/or a detection agent. The composition may contain other components as required. Examples of other components include bases, carriers, solvents, dispersants, emulsifiers, buffers, stabilizers, excipients, binders, disintegrants, lubricants, thickeners, moisturizers, colors, fragrances, and chelating agents.

The test drug of the present disclosure may be used in a kit containing a standard sample and/or a detection agent. The kit may contain an instrument, a reagent, and the like that can be used in the implementation of the testing method of the present disclosure.

### Examples

The present invention is described in detail below based on examples. However, the present invention is not limited by these examples.

### Reference Example 1. Preparation of urine samples

The urine samples used in Examples 1 and 2 below, which were obtained from subjects (6 healthy subjects and 14 prostate cancer patients (sometimes simply referred to below as "patients")), were 5 days of urine samples provided by male subjects who gave informed consent. Table 2 shows the age, prostate cancer stage, and treatment history of each subject.

**Table 2**

| ID No. | Age | Stage | Preoperative treatment history |
|---|---|---|---|
| Healthy subject 1 | 52-75 years old | - | None |
| Healthy subject 2 | | | None |
| Healthy subject 3 | | | None |
| Healthy subject 4 | | | None |
| Healthy subject 5 | | | None |
| Healthy subject 6 | | | None |
| Prostate cancer patient 1 | 62-78 years old | Stage II | None |
| Prostate cancer patient 2 | | | None |
| Prostate cancer patient 3 | | | None |
| Prostate cancer patient 4 | | | None |
| Prostate cancer patient 5 | | | None |
| Prostate cancer patient 6 | | | None |
| Prostate cancer patient 7 | | | None |
| Prostate cancer patient 8 | | | None |
| Prostate cancer patient 9 | 58-80 years old | Stage II (1 patient: stage III) | Endocrine treatment |
| Prostate cancer patient 10 | | | Endocrine treatment |
| Prostate cancer patient 11 | | | Endocrine treatment |
| Prostate cancer patient 12 | | | Endocrine treatment |
| Prostate cancer patient 13 | | | Endocrine treatment |
| Prostate cancer patient 14 | | | Endocrine treatment |

The majority of the patients were in stage II (cancer confined to the interior of the prostate gland), which is classified as the early stage of prostate cancer stages.

Three months after undergoing total prostatectomy, the patients were subjected to a PSA test and determined to be negative for prostate cancer. Urine samples collected from these patients was used as postoperative urine samples.

All urine samples were filtered through a filter with a pore size of 0.2 µm immediately after collection to remove impurities and sterilize the samples, and the resulting samples were then stored at -80°C until used for measurements.

### Example 1: Specification of prostate cancer markers using solid-phase microextraction-gas chromatography mass spectrometry

In order to reduce personal odors due to diet and genetic predisposition and odors due to occult blood, a urine sample mixture was prepared by mixing an equal volume of 5 days of urine samples from 5 patients (62-77 years old, cancer stage II, Gleason score: 7.2 (mean)). Similarly, 6 healthy subjects (52-75 years old) were divided into two groups of three subjects, and a urine sample mixture was prepared in each group by mixing an equal volume of 6 days of urine samples from 3 subjects.

When urine sample from patients after endocrine treatment is measured, 5 days of urine samples from 5 patients who underwent endocrine therapy and who had yet to undergo radical total prostatectomy (58-80 years old, 4 patients: stage II, 1 patient: stage III, Gleason score: 6.0 (mean)) were mixed.

The urine sample mixture was placed in a sealed glass container and heated to collect a large amount of volatile components, and the components were analyzed using a mass spectrometer. The compounds were adsorbed on solid-phase microextraction fibers coated with divinylbenzene/Carboxen/polydimethylsiloxane (DVB/CAR/PDMS) (Supelco, #57348-U, produced by Sigma-Aldrich) to concentrate the dilute components.

250 µL of urine sample was placed in a 2-mL glass vial and incubated at 40°C for 30 minutes. The fibers described above were then inserted and maintained for 20 minutes to adsorb the compounds. The compound-adsorbed fibers were inserted into the inlet of a GC-MS (GCMS-TQ8030, produced by Shimadzu Corporation) to desorb the compound and volatile components were measured. The measurement was performed twice for each sample. As a quality control sample for analysis, a urine sample mixture was prepared by mixing an equal volume of each of all the urine samples used in analysis, regardless of whether the source is a healthy subject or a patient, and this urine sample mixture was also analyzed together. The analytical conditions for GC-MS are as follows.

### Analysis conditions

- Analytical instrument: GCMS-TQ8030 system (produced by Shimadzu Corporation)
- Column: CP-Sil 8 CB (30 m x 0.25 mm internal diameter x 0.25 um film thickness, Agilent Technologies, Inc.)
- Mobile phase gas: Helium, 1.0 mL/min, constant flow rate
- Temperature rise conditions: 40°C (0 min) -> 40°C (5 min) -> 240°C (45 min) → 240°C (50 min)
- Injection conditions: 2 min, 240°C, splitless,
- Scan range: m/z 40-500, 0.2 scans/sec
- Ionization development temperature: 180°C
- Interface temperature: 300°C
- Ionization voltage: 70 eV.

The relative standard deviation (%RSD) per analysis was calculated for each peak, based on the height values of all peaks obtained from the QC sample analysis. Peaks having a %RSD of <30% and being 5-times higher than the negative target were set as analysis targets. Peaks that tend to be detected differently due to technical factors in the instrumental analysis process and noise peaks were excluded and 160 peaks were obtained as the target of analysis that are peaks reproducibly detected in each analysis. Subsequently, a urine sample mixture of the healthy subjects and preoperative and postoperative urine mixture samples of prostate cancer patients were each measured by GC-MS. Using the obtained peak height values as variables, principal component analysis was performed to newly obtain synthetic variables for the first and second principal components. The principal component scores for each urine mixture were plotted with the first and second principal components as axes.

Data of the samples obtained from healthy subjects and prostate cancer patients (preoperative) were plotted in different positions along the PC1 axis, indicating distinctly different profiles of volatile components. A comparison between the samples obtained from healthy subjects and those obtained from patients with prostate cancer treated with endocrine therapy (preoperative) also showed that data of the samples were plotted in different positions and had distinctly different profiles of volatile components. However, this difference was not as great as the difference between the samples obtained from healthy subjects and those obtained from prostate cancer patients (preoperative). The results of the samples obtained from patients with prostate cancer treated with endocrine therapy (preoperative) were located closer to those of the samples obtained from healthy patients along the PC1 axis. Since the value of the first principal component was positively large in the healthy subjects and negatively large in the prostate cancer patients, the first principal component was considered to be an axis that indicates health status. It can be speculated from this that the fact that the results of the samples obtained from the patients with prostate cancer treated with endocrine therapy (preoperative) were located closer to those of the samples obtained from healthy patients possibly reflects that due to the effect of endocrine treatment in suppressing cancer progression, the amount of cancer-specific volatile compounds released from the cancer tissue was reduced and the volatile compound profile became closer to the profile of volatile compounds released from the cancer tissue of healthy subjects.

Samples obtained from prostate cancer patients (postoperative), who are patients after removal of cancer, and samples obtained from patients with prostate cancer treated with endocrine therapy (preoperative) were plotted closer to those from healthy subjects in the positive direction along the PC1 axis than those from preoperative cancer patients. The presence or absence of cancer was considered to alter the profile of volatile components released into urine samples.

Similarly, the principal component scores of individual substance peaks obtained from GC-MS analysis were plotted on a PC1 axis-PC2 axis graph. From the above results, peaks with high negative first principal component scores were considered to indicate component populations that characterize prostate cancer. Eight compounds in the component populations (dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone) were specified as prostate cancer markers.

### Example 2. Quantitative analysis of volatile organic compounds in human urine samples (changes in urinary concentrations before and after the surgery)

The preoperative urine samples of five cancer patients and postoperative urine samples of the same five cancer patients were used to quantitatively analyze the concentrations of the seven target compounds specified in Example 1 (dimethyl glutarate, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone). Each urine sample was obtained by mixing an equal volume of urine samples collected 5 times. The details of the quantitative analysis method are as follows.

### Preparation of Test Solutions

### (1) Standard stock solutions

Each target compound was diluted in DMSO to concentrations of 10, 20, 50, 200, 500, 2000, 5000, 10000, and 20000 ng/mL (1, 2, 5, 20, 50, 200, 500, 1000, and 2000 ng/mL as a concentration in the sample) and the resulting dilute solutions were used as standard stock solutions.

### (2) Standard solutions

250 µL of purified water was added to 20 mL headspace vials, and 25 µL of each standard stock solution for calibration curve was added.

### (3) Sample solution and blank solution

250 µL of a sample was placed in a 20 mL headspace vial, and 25 µL of DMSO was added. A blank solution was prepared in the same manner except that TOC purified water was used in place of the sample.

### (4) Added sample solution

250 µL of a sample was placed in 20 mL headspace vials, and 25 µL of 200, 500, and 2000 ng/mL standard stock solutions were individually added.

### Device

- GC-MS: GCMS-QP2010Ultra (produced by Shimadzu Corporation)
- Autosampler: AOC-5000 Plus (produced by Shimadzu Corporation)

### SPME section

- SPME fibers: two layers of a dispersion of divinylbenzene and Carboxen and polydimethylsiloxane (DVB/CAR/PDMS)
- In-vial equilibrium temperature: 40°C
- In-vial equilibrium time: 30 min
- Extraction time: 20 min
- Desorption time: 4 min
- Fiber heat cleaning temperature: 260°C
- Fiber heat cleaning time: 10 min

### GC section

- Column: InertCap Pure-WAX, inside diameter: 0.25 mm; length: 30 m; film thickness: 0.5 um (produced by GL Sciences Inc.)
- Column temperature: 40°C (maintained for 5 min) → 5°C/min → 240°C (maintained for 5 min)
- Inlet temperature: 240°C
- Carrier gas control mode: The linear velocity is controlled so that the carrier gas flow rate is 1 mL/min.
- Sample introduction method: splitless
- Injection volume: 1 µL

### MS section

- Ionization method: EI method
- Interface temperature: 240°C
- Ion source temperature: 200°C
- Gain: Auto tuning result relative value +0.2 kV
- Mode: scan
- Scan speed: 0.2 scan/sec.

Standard solutions were individually measured in InertCap Pure WAX. After the resulting Total Ion Chromatogram (TIC) was obtained, the peaks of the target compounds were identified from the mass spectra of the detected peaks and the ion to be monitored was selected (Table 3).

**Table 3**

| Compound name | Monitored ion (m/z) |
|---|---|
| Dimethyl glutarate | Quantification: 129 |
| | Reference: 59, 100 |
| 2-Hydroxy-2-methylpropiophenone | Quantification: 105 |
| | Reference: 59, 77 |
| 2,6-di(propan-2-yl)phenol | Quantification: 165 |
| | Reference: 178, 121 |
| Dimethyl succinate | Quantification: 115 |
| | Reference: 55, 59, 114 |
| Acetophenone | Quantification: 105 |
| | Reference: 77, 120 |
| 2-Phenyl-2-propanol | Quantification: 43 |
| | Reference: 121, 77 |
| 3,5,5-Trimethyl-2-cyclohexenone | Quantification: 138 |
| | Reference: 82, 54 |

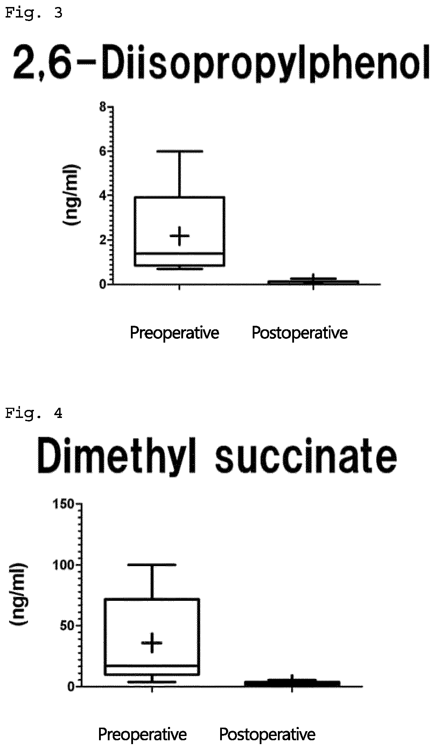

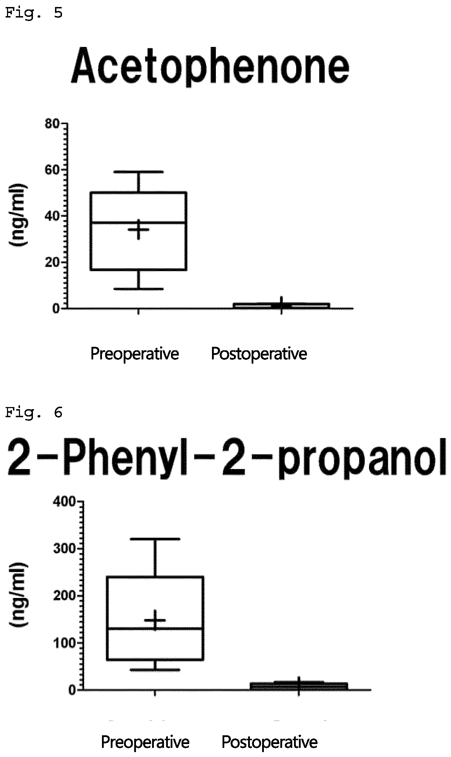

Figs. 1 to 7 show the results. Seven target compounds are components that show higher values in the preoperative samples than those in the postoperative samples. This result indicates that these compounds are derived from prostate cancer tissue.

### Example 3: Quantitative analysis of volatile organic compounds in human urine samples (comparison in urinary concentrations between healthy subjects and patients)

Four days of urine samples from 6 healthy subjects (24 samples in total) and two to five days of urine samples from 4 prostate cancer patients (13 samples in total) were used to test the eight target compounds specified in Example 1 (dimethyl glutarate, 2-hydroxy-2-methylpropiophenone, 2,6-di(propan-2-yl)phenol, dimethyl succinate, acetophenone, 2-phenyl-2-propanol, 3,5,5-trimetyl-2-cyclohexenone, and 2,6-xylidine). The quantitative analysis method used was the same as in Example 2.

The monitored ion for 2,6-xylidine, which was not selected in Example 2, is as follows.

**Table 4**

| Compound name | Monitored ion (m/z) |
|---|---|
| 2,6-Xylidine | Quantification: 121 |
| | Reference: 106, 91 |

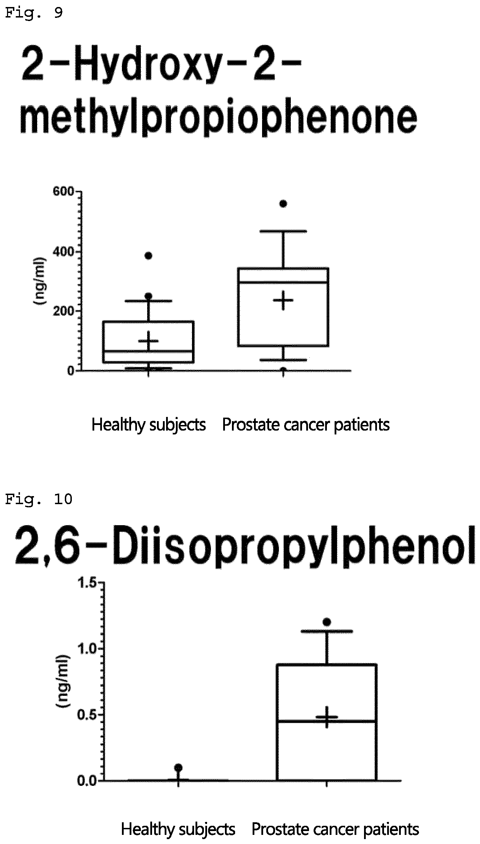

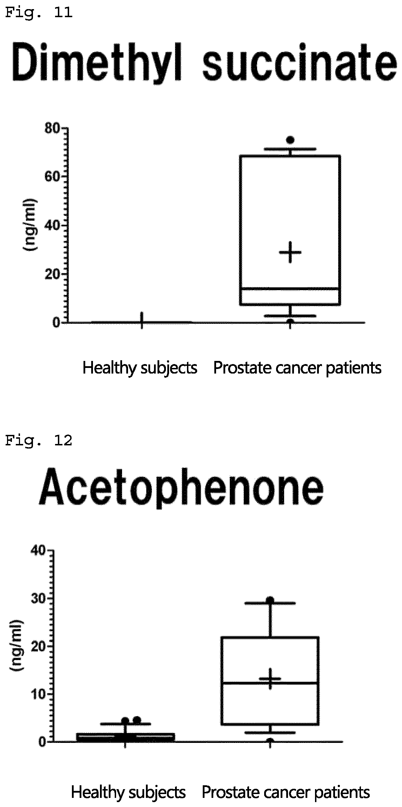

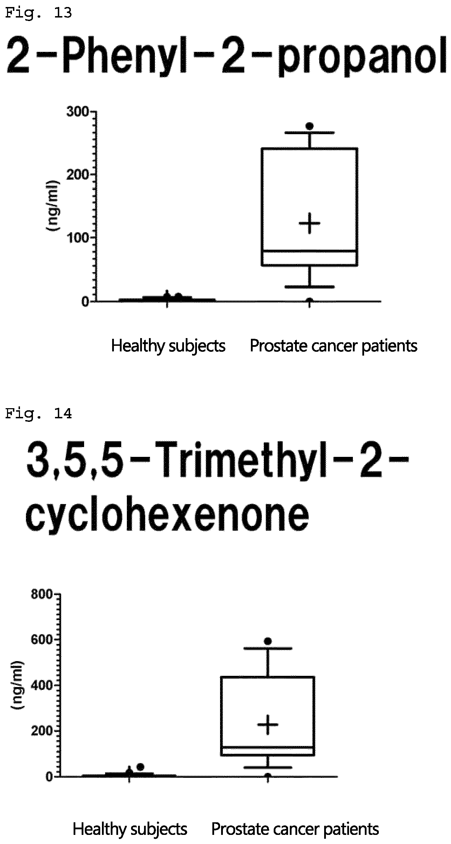

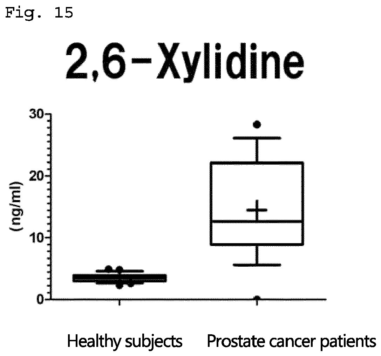

Figs. 8 to 15 show the results. Eight target compounds are components that show higher values in samples from prostate cancer patients than those in samples from healthy subjects. This result indicates that these compounds are derived from prostate cancer tissue.

## Claims

1. A method for testing for the possibility of having prostate cancer, comprising the step of
(1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

2. The method according to claim 1, further comprising the step of
(2a) determining that the subject has a high possibility of having prostate cancer when the amount or concentration of the biomarker detected in step (1) is more than or equal to a reference value A and/or
(2b) determining that the subject has a low possibility of having prostate cancer when the amount or concentration of the biomarker detected in step (1) is less than or equal to a reference value B.

3. The method according to claim 2, wherein the reference value A and the reference value B are values based on the maximum, average, percentile, or minimum value of the amount or concentration of the biomarker in body fluid samples collected from subjects determined to have no prostate cancer or subjects determined to have prostate cancer.

4. The method according to any one of claims 1 to 3,
wherein the measurement method in step (1) is gas chromatography-mass spectrometry.

5. The method according to claim 4, wherein an ion monitored in the gas chromatography-mass spectrometry is:
(A) at least one member selected from the group consisting of ions with m/z 129, ions with m/z 59, and ions with m/z 100 when dimethyl glutarate is measured;
(B) at least one member selected from the group consisting of ions with m/z 121, ions with m/z 106, and ions with m/z 91 when 2,6-xylidine is measured;
(C) at least one member selected from the group consisting of ions with m/z 105, ions with m/z 59, and ions with m/z 77 when 2-hydroxy-2-methylpropiophenone is measured;
(D) at least one member selected from the group consisting of ions with m/z 163, ions with m/z 178, and ions with m/z 121 when 2,6-di(propan-2-yl)phenol is measured;
(E) at least one member selected from the group consisting of ions with m/z 115, ions with m/z 55, ions with m/z 59, and ions with m/z 114 when dimethyl succinate is measured;
(F) at least one member selected from the group consisting of ions with m/z 105, ions with m/z 77, and ions with m/z 120 when acetophenone is measured;
(G) at least one member selected from the group consisting of ions with m/z 43, ions with m/z 121, and ions with m/z 77 when 2-phenyl-2-propanol is measured; and
(H) at least one member selected from the group consisting of ions with m/z 138, ions with m/z 82, and ions with m/z 54 when 3,5,5-trimetyl-2-cyclohexenone is measured.

6. The method according to any one of claims 1 to 5,
wherein the biomarker is two or more biomarkers.

7. The method according to any one of claims 1 to 6,
wherein the body fluid sample is a urine sample.

8. A prostate cancer biomarker in a body fluid sample, the biomarker comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

9. A prostate cancer test drug comprising a standard sample and/or a detecting agent, the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

10. A method for measuring a prostate cancer biomarker, comprising the step of
(1) measuring the amount or concentration of a biomarker in a body fluid sample collected from a subject, the biomarker being at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

11. A standard sample and/or a detecting agent for use as a prostate cancer test drug,
the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

12. Use of a standard sample and/or a detecting agent in testing for prostate cancer,
the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.

13. Use of a standard sample and/or a detecting agent in producing a prostate cancer test drug,
the standard sample comprising at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone, and
the detecting agent being for detection of at least one member selected from the group consisting of dimethyl glutarate, 2,6-xylidine, 2-hydroxy-2-methylpropiophenone, 2,6-di(propan-2-yl)phenol, dimethyl succinate, acetophenone, 2-phenyl-2-propanol, and 3,5,5-trimetyl-2-cyclohexenone.
